# EUROPEAN PATENT APPLICATION

(11) **EP 3 919 481 A1**
(43) Date of publication of application: **08.12.2021**
(21) Application number: 20747806.6
(22) Date of filing: 20.01.2020
(51) Int. Cl.: C07D 305/14, A61P 35/00, A61P 3/06, A61P 29/00, A61K 31/337, A61K 47/54

(54) **LYMPHATIC MEDIATED TRANSPORT-BASED TRIGLYCERIDE PRODRUG, AND PREPARATION METHOD THEREFOR**

(30) Priority: 01.02.2019 CN 201910103306
(71) Applicant: Shenyang Pharmaceutical University, Shenyang Liaoning 110016 (CN); Suzhou Yutai Pharmaceutical Technology Co., Ltd., Suzhou Area, 215128 (CN)
(72) Inventor: SUN, Jin, Shenyang, Liaoning 110016 (CN); HE, Zhonggui, Shenyang, Liaoning 110016 (CN); TIAN, Chutong, Shenyang, Liaoning 110016 (CN)
(74) Representative: Einsel Attorney at Law und Rechtsanwälte PartmbB
(86) International application number: PCT/CN2020/073144
(87) International publication number: WO 2020/156307

(57) **Abstract**

The present invention relates to the technical field of pharmacy, relates to a lymphatic mediated transport-based triglyceride prodrug, and specifically relates to a triglyceride prodrug of different joining chains in a lymphatic mediated transport, the preparation thereof and an application thereof in drug delivery. The present invention provides a prodrug of different connection bonds and a synthesis method therefor. The structure of the prodrug is as shown below. X, R₁, R₂, n, and m are described in the claims and description. According to the prodrug provided by the present invention, the digestion and absorption mechanism of glycerol in the gastrointestinal tract is simulated by using the structure of triglycerides, the lymphatic transport of drugs is promoted, the first pass effect is avoided, and the prodrug has the targeting property and can obviously enhance or improve the drug oral bioavailability.

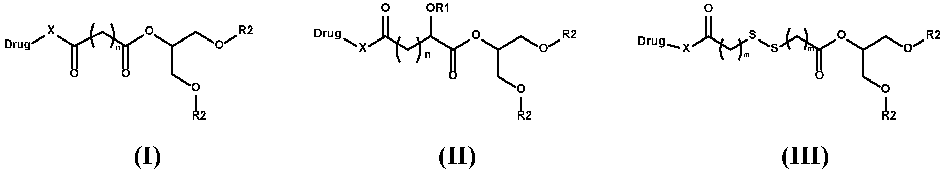

## Description

### TECHNICAL FIELD

The present invention belongs to the field of medical technologies, and relates to a triglyceride prodrug based on lymphatic-mediated transport, in particular, the triglyceride prodrug with different linkage chains for lymphatic-mediated transport, a preparation method thereof and an application thereof in drug delivery.

### BACKGROUND

Oral administration is the most convenient way of administration and has many advantages, including low treatment cost, high patient compliance, and so on. However, it also faces many problems. Some drugs have poor water solubility and poor intestinal membrane permeability. Many drugs could be metabolized by enzymes in the intestine and liver or efflux by P-glycoprotein (P-gp), resulting in low oral bioavailability of drugs. In addition, the gastrointestinal toxicity is also a major drawback of oral administration. These limiting factors are huge challenges for the development of oral administration.

The lymphatic system plays an important role in nutrient absorption, immune response, lipid physiological homeostasis, tumor metastasis and other aspects. Therefore, lymphatic transport of drugs is an effective way to improve the pharmacokinetic behavior and pharmaceutical effects of drugs. Wherein, the lymphatic transport can avoid a first pass effect and change the distribution of drugs, improving oral absorption of the drugs. Generally speaking, some drugs can enter the lymphatic circulation together with lipids by participating in the assembly of lipoproteins. These drugs need to meet a condition of high lipid solubility (LoD>5 and solubility in long-chain triglycerides > 50 mg/g). Most drugs cannot meet this condition. Therefore, by combination with prodrugs strategy, lipophilic modification of drugs is a feasible way to promote their lymphatic transport. A classic strategy is to introduce lipophilic fatty acid chains. However, this kind of prodrug is unstable under the highly active esterases in the intestine, which limits its oral absorption. Another strategy to improve the drugs lymphatic transport is to make them fully participate in the digestion, absorption and transport of lipid nutrients. The most common lipid nutrient is triglycerides. During the digestion process of triglycerides in the intestine, fatty acids in positions 1 and 3 are specifically hydrolyzed by pancreatic lipase, and fatty acid at position 2 is hardly hydrolyzed. When the fatty acid at position 2 is replaced by a drug, the obtained triglyceride-like prodrug can mimic the digestion process of triglycerides in the intestine, i.e., enter intestinal epithelial cells in the form of 2-monoglyceride-like prodrug for re-esterification, participate in the assembly of lipoproteins, and thus promote the lymphatic transport. In this process, pancreatic lipase plays an important role in the hydrolysis of fatty acids at positions 1, 3, and the generated monoglyceride prodrugs can form micelles with bile salt phospholipids etc., and then promote their absorption into intestinal epithelial cells. Therefore, investigation of the affinity of the structure of the prodrug to pancreatic lipase is of great significance.

Predecessors have done a lot of work on triglyceride prodrugs, such as directly linking a carboxyl-containing drug to hydroxyl groups of a triglyceride skeleton (Pharmazie, 2005, 60(2), 110-114; Journal of Medicinal Chemistry, 1979, 22(6), 683-687), and some researchers inserted carbon chains of different chain lengths between the triglyceride skeleton and the parent drug as a linking bridge (J. Androl. 2003, 24, 716-720; Arch. Pharm. 1995, 328, 271-276), but this prodrug released the parent drug very slowly and incompletely, resulting in no significant improvement in oral bioavailability. Professor Christopher. J. H. Porter introduced a self-eliminating bridge between the triglyceride skeleton and the parent drug testosterone, which promoted the lymphatic transport of the prodrug, and also allowed it to quickly release the parent drug after entering the blood circulation, thereby improving the oral bioavailability and promoting the efficacy (Angewandte Chemie, 2016, 55(44):13904-13909). For oral chemotherapy, in addition to improving the oral bioavailability of a drug, the specific release of the drug at the tumor site is also a top priority. Therefore, it is very meaningful to introduce a sensitive bond that can specifically respond to tumor microenvironments for triglyceride-like prodrug.

Triglycerides are digested by pancreatic lipase in the intestine, enter intestinal epithelial cells in the form of monoglyceride and participate in the assembly of lipoproteins, and are then transported into the blood through the lymphatic transport. The special absorption physiological process of the triglycerides provides important ideas for the design of prodrugs. There are many prodrug designs with triglyceride as a skeleton, but there is no research to systematically discuss the relationship between the structure of the prodrug and the affinity of pancreatic lipase, and the effect of its affinity on the oral absorption of the prodrug. Meanwhile, some researchers have explored triglycerides as a carrier for lymphatic delivery of anti-tumor drugs, but there are few studies on oral administration of triglyceride prodrugs against solid tumors.

### SUMMARY

The first object of the present invention is to design triglyceride-like prodrugs based on the natural triglyceride lymphatic transport mechanism to promote the lymphatic transport of poorly soluble drugs and improve their oral availability. On this basis, different linking bridges are introduced: 1) a straight carbon chain linkage bridge, as a control; 2) a carbon chain linking bridge with an α-position substituent, used to explore the effect of the affinity of the prodrug structure to pancreatic lipase; 3) a reduction-sensitive linking bridge, which can make the prodrug specifically release at the target site while promoting the oral absorption of anti-tumor drugs, thereby improving the antitumor efficacy and reducing the toxicity.

The second object of the present invention is to provide a method for synthesizing prodrugs connected by the aforementioned different linking bonds.

The third object of the present invention is to provide and compare the effects of the prodrugs with the aforementioned different linking bonds on the oral absorption of drugs.

In order to achieve the above object, the present invention provides compounds represented by formula (I) (II) (III), geometric isomers, and pharmaceutically acceptable salts, hydrates and solvates thereof: 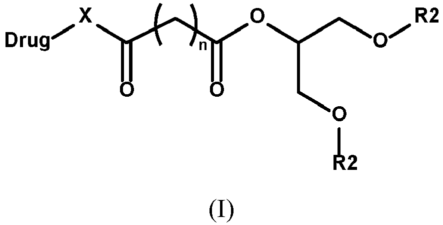 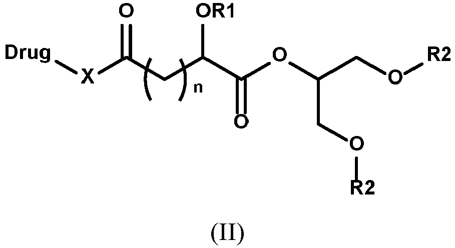 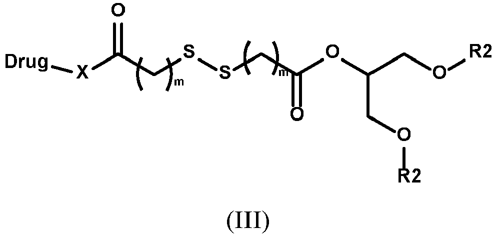 wherein
X is NH, O, S;
"Drug" is a poorly soluble drug containing hydroxyl, amino or sulfhydryl;
n=1-10; m=1-3;
R1 is C1-C6 acyl, preferably acetyl, propionyl, isopropionyl, pivaloyl, benzoyl; and
R2 is C2-C24 saturated or unsaturated aliphatic acyl.

Preferably,
X is O;
"Drug" is a poorly soluble drug containing hydroxyl, amino or sulfhydryl;
n=1-5; m=1-3; and
R2 is C10-C24 saturated or unsaturated aliphatic acyl.

Among the compounds with the structures represented by the general formulas of the present invention: the poorly soluble drugs containing amino, hydroxyl or sulfhydryl include anti-tumor drugs, hormone drugs, hypolipidemic drugs, non-steroidal anti-inflammatory drugs and other poorly soluble drugs. The anti-tumor drugs are selected from taxanes, anthraquinones, nucleosides, etc. The hormone drugs are selected from testosterone, progesterone, etc. The hypolipidemic drugs are statins. The non-steroidal anti-inflammatory drugs and other poorly soluble drugs are selected from halofantrine, griseofulvin, cyclosporine A and their derivatives.

Taking docetaxel as an example, the present invention provides a preferred structure of the compound represented by the general formula (I):

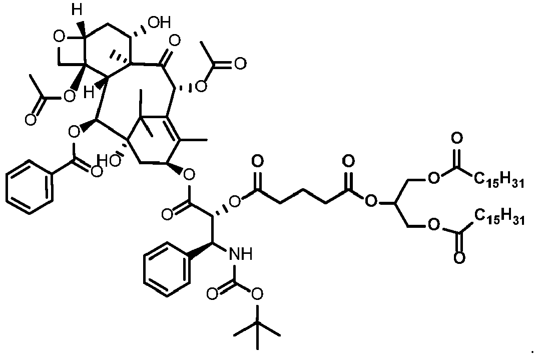

A preferred structure of the compound represented by the general formula (II) is:

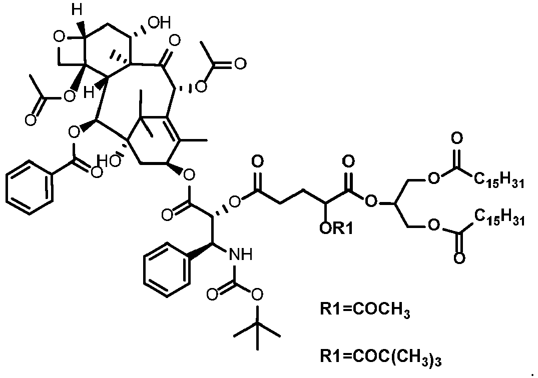

A preferred structure of the compound represented by the general formula (III) is:

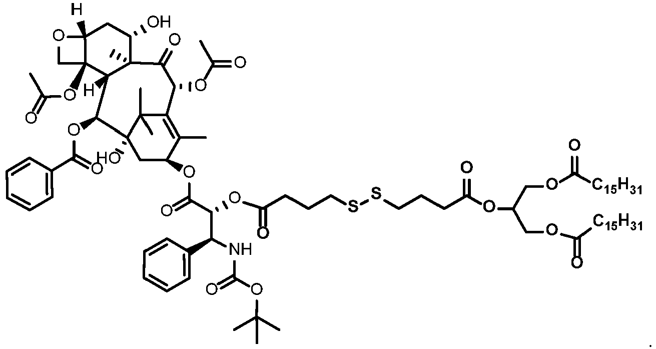

The compounds of the present invention may be prepared by the following methods:

A method for preparing the compound of general formula (I) includes:
(1) generating aliphatic acyl chloride from aliphatic acid in the presence of thionyl chloride; then reacting the aliphatic acyl chloride with 1,3-dihydroxyacetone under the catalyst of triethylamine to obtain a reactant; and preparing 1,3-diglyceride by catalytically hydrogenating the reactant with sodium borohydride; and
(2) reacting the 1,3-diglyceride with straight-chain aliphatic dibasic acid or aliphatic dibasic acid anhydride in the presence of pyridine to obtain a reactant; and generating a target prodrug by esterifying or ammoniating the reactant with an active drug in the presence of 4-dimethylaminopyridine (DMAP).

The aliphatic acid in step (1) is C2-C24 saturated or unsaturated aliphatic acid.

The straight-chain aliphatic dibasic acid in step (2) is selected from: malonic acid, succinic acid, glutaric acid, adipic acid, pimelic acid, suberic acid, azelaic acid, and sebacic acid.

A method for preparing the compound of formula (II) includes:
(1) generating aliphatic acyl chloride from the aliphatic acid in the presence of thionyl chloride; reacting the aliphatic acyl chloride with 1,3-dihydroxyacetone under the catalyst of triethylamine to obtain a reactant; and preparing 1,3-diglyceride by catalytically hydrogenating the reactant with sodium borohydride; and
(2) converting an amino group into a hydroxyl group by reacting L-glutamic acid-γ-benzyl ester with sodium nitrite in a mixed solvent of glacial acetic acid and water; esterifying with different alkanoyl chlorides or alkanoic acid anhydrides under the catalysis of 4-dimethylaminopyridine (DMAP) to obtain a product; esterifying the product and 1,3-diglyceride in the presence of DMAP and 1-(3-dimethylaminopropyl)-3-ethylcarbodiimide hydrochloride (EDCI) to obtain a product; catalytically reducing the product with hydrogen; and obtaining a target prodrug by performing an esterified or ammoniated reaction on the reduced product and an active drug in the presence of DMAP and EDCI.

The aliphatic acid in step (1) is selected from: C2-C24 saturated or unsaturated aliphatic acid.

The alkanoyl chloride or alkanoic acid anhydride in step (2) is selected from: acetic acid anhydride, propionic acid chloride, isopropanoyl chloride, and pivaloyl chloride.

A method for preparing the compound of formula (III) includes:
(1) generating aliphatic acyl chloride from the aliphatic acid in the presence of thionyl chloride; then reacting the aliphatic acyl chloride with 1,3-dihydroxyacetone under the catalyst of triethylamine to obtain a reactant; and preparing 1,3-diglyceride by catalytically hydrogenating the product with sodium borohydride; and
(2) reacting the 1,3-diglyceride with different aliphatic dithio-dibasic acid anhydrides in the presence of triethylamine to obtain a reactant; and acquiring a target prodrug by esterifying or ammoniating the reactant and an active drug in the presence of DMAP and EDCI.

The aliphatic acid in step (1) is selected from: C2-C24 saturated or unsaturated aliphatic acid.

The aliphatic dithio-dibasic acid anhydride is selected from: 2,2'-dithiodiacetic acid, 3,3'-dithiodipropionic acid, and 4,4'-dithiodibutyric acid.

Taking docetaxel as an example, each of the prodrugs of the present invention is connected with a triglyceride skeleton and is designed and synthesized as a docetaxel triglyceride prodrug linked by different linking chains (a straight carbon-chain linking bridge, a carbon-chain linking bridge with α-position substituent and a reduction-sensitive dithio bond). The specific synthesis route and method are as follows.

Synthesis of 1,3-palmitic acid diglyceride (1,3-DG): palmitic acid is dissolved in dioxane and reacted with thionyl chloride to obtain palmitoyl chloride. The palmitoyl chloride is reacted with 1,3-dihydroxyacetone in the presence of triethylamine, to obtain 1,3-palmitoyl-2-carbonylpropane, which is further hydrogenated with sodium borohydride to obtain 1,3-DG. 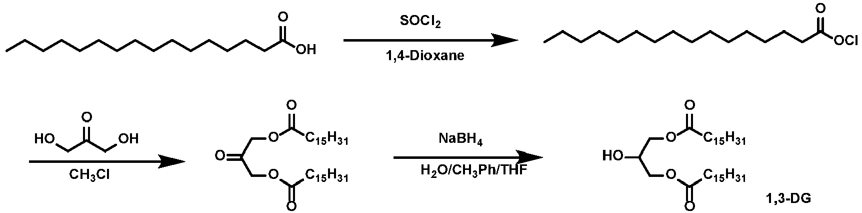
(a) Synthesis of a triglyceride prodrug with a straight carbon-chain linking bridge: glutaric acid anhydride is reacted with 1,3-DG in the presence of 4-dimethylaminopyridine (DMAP) and pyridine to obtain an intermediate 1; and the compound 1 and docetaxel are reacted under the catalyst of 4-dimethylaminopyridine (DMAP) and 1-(3-dimethylaminopropyl)-3-ethylcarbodiimide hydrochloride (EDCI) to obtain the target prodrug. 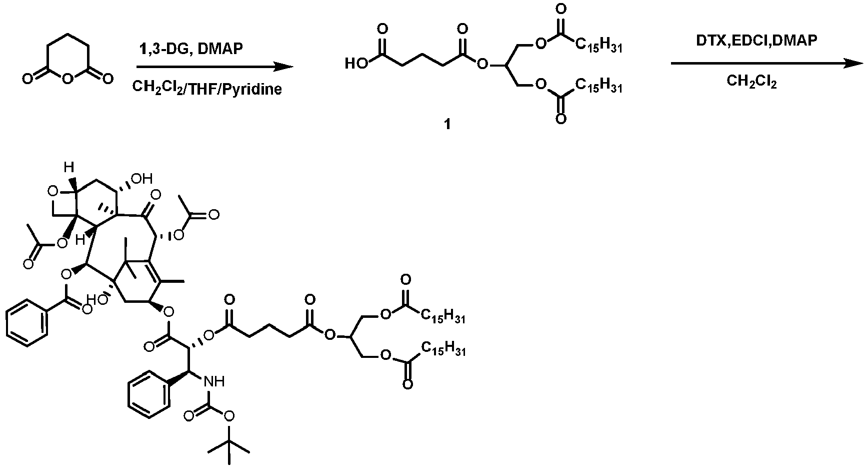
(b) Synthesis of a triglyceride prodrug with a carbon chain linking bridge having α-position substituent: L-glutamic acid-γ-benzyl ester is dissolved in a mixed solvent of glacial acetic acid and water, and reacted with sodium nitrite to obtain a compound 2; and the compound 2 is reacted with acetic anhydride or pivaloyl chloride in the presence of 4-dimethylaminopyridine (DMAP) to obtain a compound 3 or 4. 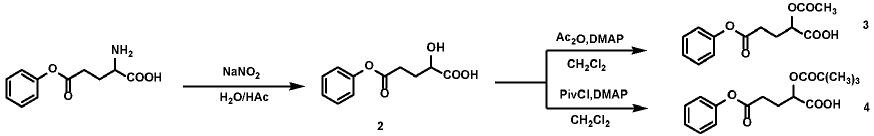
   Under the catalysis of 4-dimethylaminopyridine (DMAP) and 1-(3-dimethylaminopropyl)-3-ethylcarbodiimide hydrochloride (EDCI), the compound 3 or 4 is esterified with 1,3- DG to obtain an intermediate product 5 or 6, which is catalytically reduced with hydrogen to obtain a compound 7 or 8, and is esterified with docetaxel in the presence of 4-dimethylaminopyridine (DMAP) and 1-(3-dimethylaminopropyl)-3-ethylcarbodiimide hydrochloride (EDCI) to obtain the target prodrug. 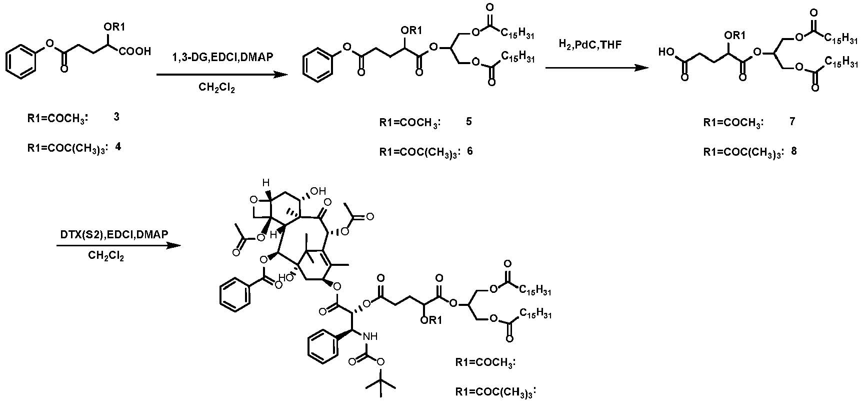
(c) Synthesis of a triglyceride prodrug with a reduction-sensitive linking bridge: dithiodibutyric acid is dissolved in an appropriate amount of acetic anhydride, and stirred at room temperature to obtain dithiodibutyric acid anhydride; the dithiodibutyric acid anhydride is reacted with 1,3-DG in the presence of triethylamine and 4-dimethylaminopyridine (DMAP) to obtain an intermediate product 9; and the intermediate product 9 is esterified with docetaxel in the presence of 4-dimethylaminopyridine (DMAP) and 1-(3-dimethylaminopropyl)-3-ethylcarbodiimide hydrochloride (EDCI) to obtain the target prodrug.

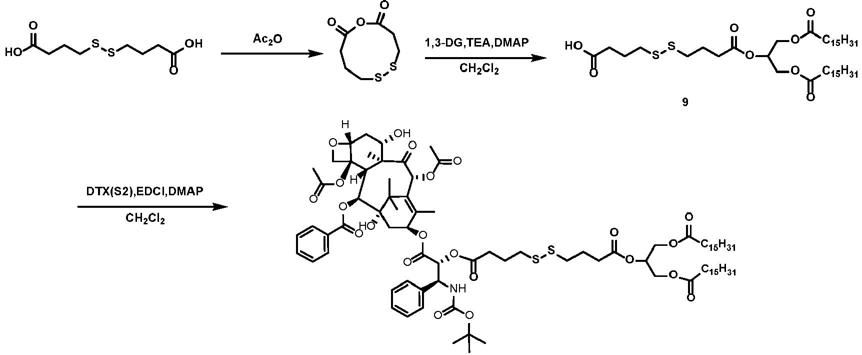

In the general formula (II) of the present invention, a substituent is introduced at the α-position of the linking bridge between the triglyceride and the drug, and structural steric hindrance is used to investigate the affinity of pancreatic lipase, and then to investigate the influence of intestinal digestion on the oral absorption of the prodrug.

The compound represented by the general formula (III) of the present invention may be used for the oral target delivery of an anti-tumor drug. Through the introduction of dithio bonds, the specific release of the prodrug at the tumor site is enhanced while the triglyceride-like structure improves oral absorption, thereby increasing the efficiency and reducing the toxicity.

According to the present invention, a series of docetaxel triglyceride prodrugs with different linking bridges are designed and synthesized by using docetaxel as a model drug, and the relationship between the structure of the prodrug and intestinal digestion is investigated by using a simulated intestinal digestion experiment. An *in vivo* intestinal perfusion experiment is used to investigate the membrane permeability of the prodrug. A rat pharmacokinetic experiment is used to compare the oral absorption of the prodrug. Further, the pharmacodynamic investigation is performed to find an optimal anti-tumor triglyceride prodrug structure.

In order to design a more reasonable triglyceride oral prodrug, the present invention introduces a substituent at the α-position of the glyceride for the first time to explore the influence of the affinity of the prodrug and pancreatic lipase on oral absorption. The investigation results showed that a straight linking bridge without branched chain at the α-position is more conducive to the lipolysis and digestion of the intestinal pancreatic lipase to the triglyceride prodrug and promotes oral absorption, while a linking bridge with a branched chain is not conducive to the lipolysis and digestion of the intestinal pancreatic lipase to the triglyceride prodrug, the greater of the steric hindrance is, the less conducive to oral absorption is. In order to design a triglyceride chemotherapeutic prodrug that can specifically inhibit solid tumors, the present invention introduces dithio bonds between the triglyceride skeleton and the anti-tumor drug for the first time so as to promote the specific release of a parent drug at the tumor site, thereby enhancing the efficacy and reducing the toxicity. The present invention provides reasonable opinions for the structural design of triglyceride prodrugs, and at the same time provides a solution for solving the problems of low oral bioavailability, poor efficacy, and high system toxicity of antitumor drugs.

**The present invention has the following advantages.**
1. The present invention designs a triglyceride-like prodrug based on an absorption mechanism of natural triglycerides, which promotes the lymphatic transport of the drug, avoids the first-pass effect, and further improves oral absorption.
2. The present invention demonstrates the influence of the intestinal digestion process on the absorption of triglyceride prodrugs by linking the triglyceride skeleton and the poorly soluble drug via the carbon-chain linking bridge with α-position substituent.
3. The present invention uses a reduction-sensitive dithio bond linking bridges to link the triglyceride skeleton and the poorly soluble drug, promoting the oral absorption of the drug and specifically release the parent drug at the target site at the same time, thereby increasing the efficiency and reducing the toxicity.
4. The present invention broadens the application prospects of oral anti-tumor drugs, making oral chemotherapy possible.

### BRIEF DESCRIPTION OF THE DRAWINGS

FIG. 1 is a graph of the gastrointestinal stability of four docetaxel triglyceride prodrugs.
FIG. 2 is a graph of the plasma stability of four docetaxel triglyceride prodrugs.
FIG. 3 is an in vitro reduction-sensitive release graph of docetaxel triglyceride prodrugs synthesized in Examples 1 and 4 of the present invention.
FIG. 4 is a graph of in vivo intestinal perfusion absorption rate constants of a docetaxel triglyceride prodrug and a docetaxel solution.
FIG. 5 is a blood concentration - time graph of a docetaxel triglyceride prodrug and a docetaxel solution.
FIG. 6 is a cytotoxicity diagram of docetaxel triglyceride prodrugs synthesized in Examples 1 and 4 of the present invention and a docetaxel solution.
FIG. 7 is an in vivo anti-tumor experiment diagram of a docetaxel triglyceride prodrug and a docetaxel solution.
FIG. 8 is a gastrointestinal HE staining diagram of the docetaxel triglyceride prodrug synthesized in Example 4 of the present invention and a docetaxel solution.

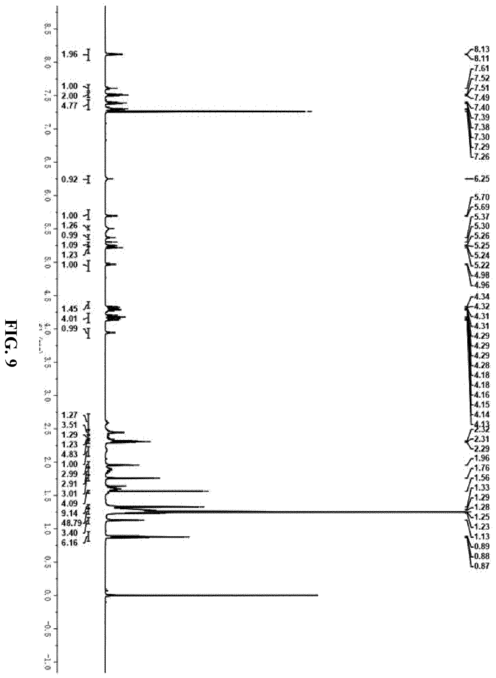

FIG. 9 is a ¹HNMR spectrum of the docetaxel triglyceride prodrug (DTX-5C-TG) synthesized in Example 1 of the present invention.

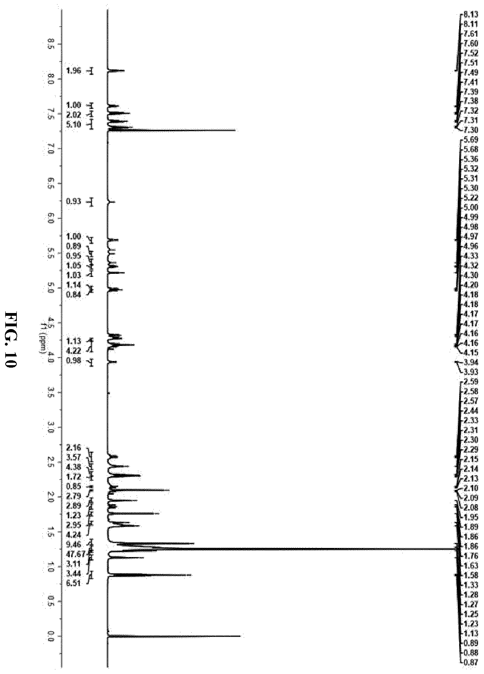

FIG. 10 is a ¹HNMR spectrum of a docetaxel triglyceride prodrug (DTX-5C(Et)-TG) synthesized in Example 2 of the present invention.

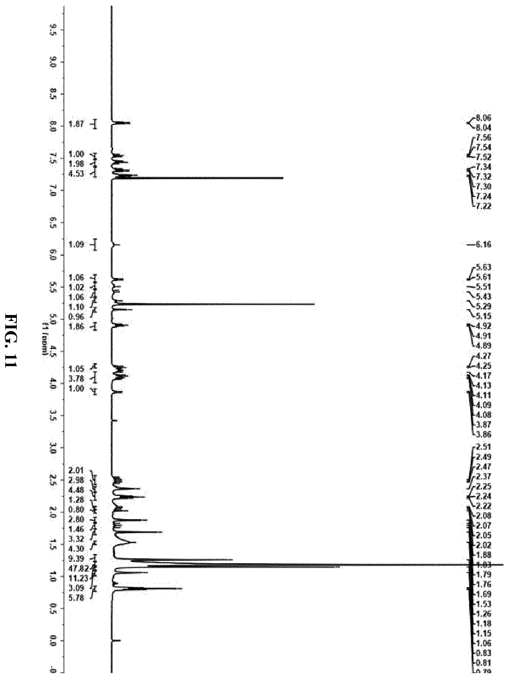

FIG. 11 is a ¹HNMR spectrum of a docetaxel triglyceride prodrug (DTX-5C(Piv)-TG) synthesized in Example 3 of the present invention.

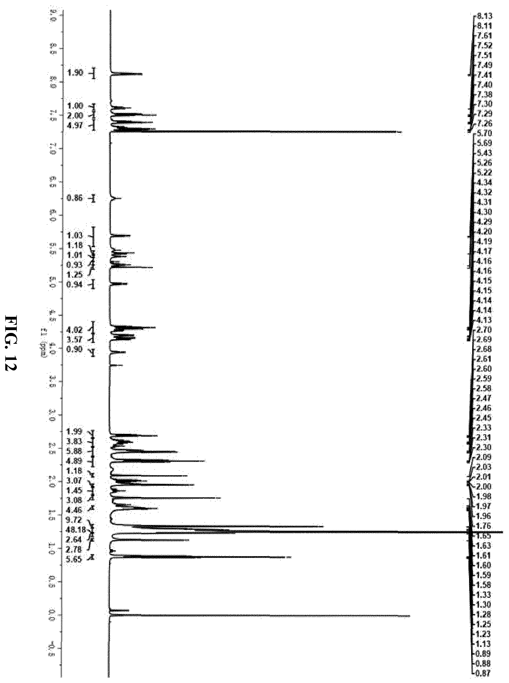

FIG. 12 is a ¹HNMR spectrum of the docetaxel triglyceride prodrug (DTX-S-S-TG) synthesized in Example 4 of the present invention.

### DETAILED DESCRIPTION

The present invention is further illustrated by the following examples, but is not limited thereto. The prodrugs are described as follows.

### Example 1 Preparation of docetaxel-C5-triglyceride prodrug (DTX-5C-TG)

The structure is as follows:

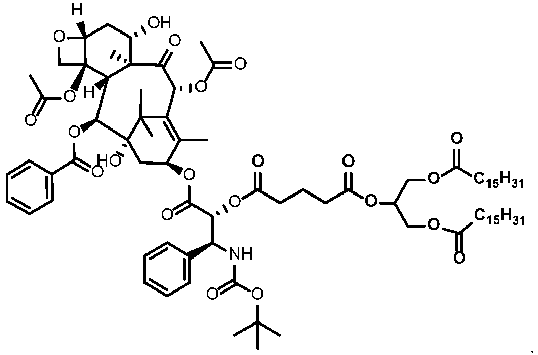

10.25 g (40 mmol) of palmitic acid is dissolved in 100 ml of anhydrous dioxane, added with 3 drops of N,N-dimethylformamide, and added with 4.9 g (41 mmol) of thionyl chloride dropwise. After the addition is completed, the resultant is vacuumized and protected with nitrogen, and refluxed at 110°C for 4 h. The reaction solution is concentrated under reduced pressure to obtain pale yellow oil. 1.75 g (20 mmol) of 1,3-dihydroxyacetone is dissolved in 100 ml of anhydrous dichloromethane, added with 5 ml (40 mmol) of triethylamine, and quickly dropwise added with the abovementioned oil under ice-bath; and after the addition is completed, the resultant is vacuumized and protected with nitrogen, and reacted at room temperature overnight. The reaction solution is concentrated, and washed with re-distilled water for 2 times. An aqueous layer is extracted with chloroform, and an organic layer is washed with saturated brine one time. The organic layer is dried over anhydrous sodium sulfate, and a solvent is removed by rotary evaporation. A crude product is recrystallized with dichloromethane and diethyl ether (1 : 1) to obtain a white solid. 2.9 g (5 mmol) of the above white solid is dissolved in a 100 ml of mixed solvent (THF : benzene : water is 10 : 2 : 1), added with 0.3 g (7.8 mmol) of sodium borohydride, reacted at 5°C for 30 min, added with 0.9 ml of glacial acetic acid till the reaction stops. The reaction solution is mixed with 80 ml of chloroform, washed with re-distilled water one time, with 4% of sodium bicarbonate solution one time and with saturated brine one time, and dried over anhydrous sodium sulfate; and the solvent is removed by rotary evaporation. The crude product is recrystallized with n-hexane and ethyl acetate (97 : 3) to obtain a pure product of 1,3-dipalmitate glyceride.

500 mg (0.88 mmol) of 1,3-dipalmitate glyceride is dissolved in 10 ml of anhydrous dichloromethane, added with 10 ml of THF, 104 mg (0.9 mmol) of DMAP and 200 mg (1.75 mmol) of glutaric acid anhydride, and slowly added dropwise with 10 ml of anhydrous pyridine. The resultant is vacuumized and protected with nitrogen, and reacted at room temperature for 48 hours. After the reaction is completed, the reaction solution is washed with 0.1 N hydrochloric acid solution one time; an organic layer is dried over anhydrous sodium sulfate; the solvent is removed by rotary evaporation; and a white solid, i.e., a compound 10 is obtained by column chromatography. 560 mg (0.83 mmol) of compound 10 is dissolved in 30 ml of anhydrous dichloromethane, added with 175 mg (0.9 mmol) of EDCI, 45 mg (0.36 mmol) of DMAP, and 803 mg (1 mmol) of docetaxel. The resultant is vacuumized and protected with nitrogen, and reacted at room temperature for 48 hours. The solvent is removed by rotary evaporation, and a white solid is obtained by column chromatography.
¹H NMR (400 MHz, CDCl₃) δ 8.12 (d, J = 7.5 Hz, 2H), 7.61 (t, J = 7.5 Hz, 1H), 7.51 (t, J = 7.6 Hz, 2H), 7.43 - 7.24 (m, 5H), 6.25 (s, 1H), 5.70 (d, 1H), 5.50 (s, 1H), 5.37 (s, 1H), 5.31 (m, 1H), 5.21 (s, 1H), 4.97 (m, 1H), 4.33 (d, 1H), 4.20-4.17 (m, 4H), 3.94 (d, J = 6.9 Hz, 1H), 2.59 (m, 1H), 2.45 (s, 3H), 2.38 (m, 1H), 2.32 (dt, 2H), 2.31 (q, 4H), 2.18 (m, 1H), 1.96 (s, 3H), 1.88 (m, 2H), 1.76 (s, 3H), 1.59 (m, 4H), 1.33 (s, 9H) 1.30-1.24 (m , 48H), 1.23 (s, 3H), 1.13 (s, 3H), 0.88 (t, J = 6.8 Hz, 6H). ESI-HRMS: Calcd. For C₈₃H₁₂₆NO₂₁ [M + H⁺] 1472.8811 found 1472.8816.

### Example 2 Preparation of docetaxel-C5(Et)-triglyceride prodrug (DTX-5C(Et)-TG)

The structure is as follows:

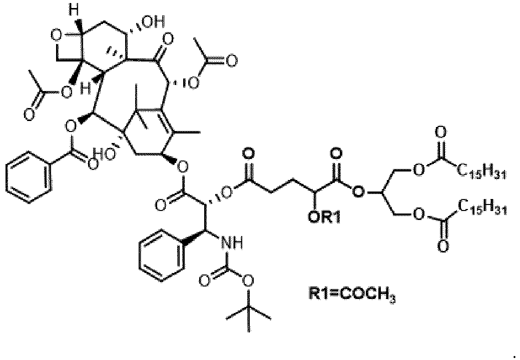

A method for preparing 1,3-dipalmitate glyceride is the same as that in Example 1. 1.5 g (6.33 mmol) of L-glutamic acid-γ-benzyl ester is dissolved in 10 ml of acetic acid, added with 10 ml of water, stirred under an ice bath for 20 min, added with 3.45 g (50 mmol) of sodium nitrite, reacted under the ice bath for 2 h, and then reacted at room temperature overnight. The reaction solution is extracted with ethyl acetate, wherein an organic layer is washed with water one time. The organic layers are combined, and dried over anhydrous sodium sulfate. The solvent is removed by rotary evaporation to obtain a compound 2. 1 g of compound 1 is again dissolved in 30 ml of anhydrous dichloromethane, added with 100 mg (0.8 mmol) of DMAP, added dropwise with 0.6 g (5.88 mmol) of acetic anhydride. After the addition is completed, the resultant is vacuumized and protected with nitrogen, and reacted at room temperature overnight. The solvent is removed by rotary evaporation, and yellow oily liquid, i.e., a compound 3 is obtained by column chromatography. 500 mg of compound 3 is dissolved in anhydrous dichloromethane, added with 200 mg (1.6 mmol) of DMAP, 700 mg (3.66 mmol) of EDCI, and 900 mg (1.58 mmol) of 1,3-dipalmitate glyceride. The resultant is vacuumized and protected with nitrogen, and reacted at room temperature for 48 h. The solvent is removed by rotary evaporation, and a compound 5 is obtained by column chromatography. 600 mg of compound 5 is dissolved in 30 ml of THF, added with 60 mg of palladium on carbon, and reacted in the presence of hydrogen at room temperature for 4 h; the palladium on carbon is filtered off; and a compound 7 is obtained by removing the solvent by rotary evaporation. 600 mg of compound 7 is dissolved in 30 ml of anhydrous dichloromethane, and added with 45 mg (0.36 mmol) of DMAP, 175 mg (0.91 mmol) of EDCI, and 803 mg (1 mmol) of docetaxel. The resultant is vacuumized and protected with nitrogen, and reacted at room temperature for 48 h. The solvent is removed by rotary evaporation, and a white solid is obtained by column chromatography.
¹H NMR (400 MHz, CDCl₃) 8.12 (d, J = 7.5 Hz, 2H), 7.61 (t, J =14.1 Hz, 1H), 7.51 (t, J = 7.6 Hz, 2H), 7.43 - 7.24 (m, 5H), 6.23 (s, 1H), 5.69 (d, 1H), 5.49 (s, 1H), 5.36 (s, 1H), 5.31 (m, 1H), 5.21 (s, 1H), 4.99 (m, 1H), 4.97 (m, 1H), 4.33 (d, 1H), 4.20-4.18 (m, 4H), 3.94 (d, J = 6.8 Hz, 1H), 2.57 (m, 2H), 2.44 (s, 3H), 2.31 (q, 4H), 2.28 (dt, 2H), 2.15 (m, 1H), 2.10 (s, 3H) 1.95 (s, 3H), 1.87 (m, 1H), 1.76 (s, 3H), 1.59 (m, 4H), 1.33(s, 9H) 1.30-1.24 (m , 48H), 1.23 (s, 3H), 1.13 (s, 3H), 0.88 (t, J = 6.8 Hz, 6H). ESI-HRMS: Calcd. For C₈₅H₁₂₇NNaO₂₃ [M + Na⁺] 1552.8742 found 1552.8691.

### Example 3 Preparation of docetaxel-C5(Piv)-triglyceride prodrug (DTX-5C-(Piv)-TG)

The structure is as follows:

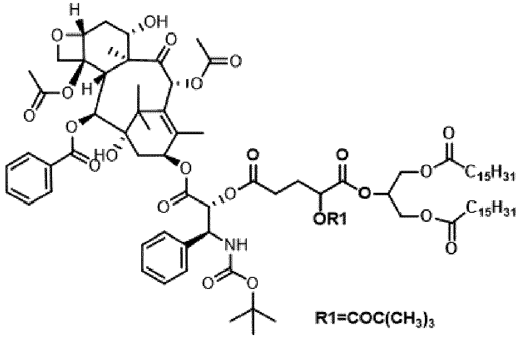

A method for preparing 1,3-dipalmitate glyceride is the same as that in Example 1. 1.5 g (6.33 mmol) of L-glutamic acid-γ-benzyl ester is dissolved in 10 ml of acetic acid, added with 10 ml of water, stirred under an ice bath for 20 min, added with 3.45 g (50 mmol) of sodium nitrite, reacted under the ice bath for 2 h, and then reacted at room temperature overnight. The reaction solution is extracted with ethyl acetate, wherein an organic layer is washed with water one time. The organic layers are combined, and dried over anhydrous sodium sulfate. A solvent is removed by rotary evaporation to obtain a compound 2. Then, 1 g of compound 1 is again dissolved in 30 ml of anhydrous dichloromethane, added with 100 mg (0.8 mmol) of DMAP, and added dropwise with 770 mg (6.28 mmol) of pivaloyl chloride. After the addition is completed, the resultant is vacuumized and protected with nitrogen, and reacted at room temperature overnight. The solvent is removed by rotary evaporation, and a compound 4 is obtained by column chromatography. 500 mg of compound 3 is dissolved in anhydrous dichloromethane, and added with 200 mg (1.6 mmol) of DMAP, 700 mg (3.66 mmol) of EDCI, and 900 mg (1.58 mmol) of 1,3-dipalmitate glyceride. The resultant is vacuumized and protected with nitrogen, and reacted at room temperature for 48 h. The solvent is removed by rotary evaporation, and a compound 6 is obtained by column chromatography. 600 mg of the white solid (i.e., the compound 6) is dissolved in 30 ml of THF, added with 60 mg of palladium on carbon, and reacted in the presence of hydrogen at room temperature for 4 h; the palladium on carbon is filtered off; and a compound 8 is obtained by removing the solvent by rotary evaporation. 600 mg of compound 7 is dissolved in 30 ml of anhydrous dichloromethane, and added with 45 mg (0.36 mmol) of DMAP, 175 mg (0.91 mmol) of EDCI, and 803 mg (1 mmol) of docetaxel. The resultant is vacuumized and protected with nitrogen, and reacted at room temperature for 48 h. The solvent is removed by rotary evaporation, and a white solid is obtained by column chromatography.
¹H NMR (400 MHz, CDCl₃) δ 8.05 (d, J = 7.4 Hz, 2H), 7.53 (dd, J = 14.1 Hz, 1H), 7.44 (t, J = 7.6 Hz, 2H), 7.39 - 7.17 (m, 5H), 6.16 (s, 1H), 5.62 (d, 1H), 5.51 (s, 1H), 5.44 (d, J = 14.5 Hz, 1H), 5.29 (m, J = 14.5 Hz, 1H), 5.15 (s, 1H), 4.92 (m, 1H), 4.90 (m, 1H), 4.26 (d, 1H), 4.17-4.06 (m, 4H), 3.87 (d, J =7.1 Hz, 1H), 2.50 (m, 2H), 2.37 (s, 3H), 2.24 (q, 4H), 2.07 (dt, 2H), 2.02 (m, 1H), 1.88 (s, 3H), 1.79 (m, 1H), 1.69 (s, 3H), 1.53 (m, 4H), 1.26 (s, 9H) 1.20-1.18 (m, 48H), 1.15 (s, 3H), 1.14 (m, 9H), 1.06 (s, 3H), 0.83 (t, J = 6.8 Hz, 6H). ESI-HRMS: Calcd. For C₈₈H₁₃₄NO₂₃ [M +H⁺] 1572.9291 found 1572.9341.

### Example 4 Preparation of docetaxel-dithio-triglyceride prodrug (DTX-S-S-TG)

The structure is as follows:

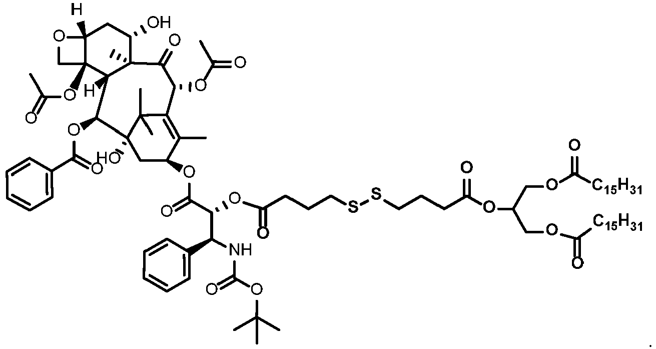

A method for preparing 1,3-dipalmitate glyceride is the same as that in Example 1. 2.1 g (8.8 mmol) of 4,4'-dithiodibutyric acid is dissolved in 9 ml of acetic anhydride, and reacted at room temperature for 2 h; a solvent is concentrated by rotary evaporation, and redissolved in 30 ml of anhydrous dichloromethane, and added with 845 mg (8.4 mmol) of triethylamine; and the resultant is vacuumized and protected with nitrogen, and reacted at room temperature for 48 hours. The solvent is removed by rotary evaporation, and a compound 9 is obtained by column chromatography. 560 mg (0.83 mmol) of compound 13 is dissolved in 30 ml of anhydrous dichloromethane, and added with 175 mg (0.9 mmol) of EDCI, 45 mg (0.36mmol) of DMAP, and 803 mg (1 mmol) of docetaxel. The resultant is vacuumized and protected with nitrogen, and reacted at room temperature for 48 hours. The solvent is removed by rotary evaporation, and a white solid is obtained by column chromatography.
¹H NMR (400 MHz, CDCl₃) δ 8.12 (d, J = 7.5 Hz, 2H), 7.62 (t, J = 7.5 Hz, 1H), 7.55 (t, J = 7.6 Hz, 2H), 7.44 - 7.24 (m, 5H), 6.23 (s, 1H), 5.69 (d, 1H), 5.48 (s, 1H), 5.38 (s, 1H), 5.34 (m, 1H), 5.25 (s, 1H), 4.97 (m, 1H), 4.30 (d, 4H), 4.20-4.17 (m, 4H), 3.94 (d, J = 6.8 Hz, 1H), 2.70 (m, 2H), 2.58 (m, 4H), 2.47 (m, 2H), 2.42 (s, 3H), 2.31 (q, 4H), 2.18 (m, 1H), 1.96 (s, 3H), 1.85 (m, 2H), 1.76 (s, 3H), 1.59 (m, 4H), 1.31 (s, 9H) 1.30-1.24 (m, 48H), 1.23 (s, 3H), 1.13 (s, 3H), 0.88 (t, J = 6.8 Hz, 6H). ESI-HRMS: Calcd. For C₈₆H₁₃₂NO₂₁S₂ [M + H⁺] 1578.8709 found 1578.8727.

A specific synthetic route of the above prodrugs is as follows:

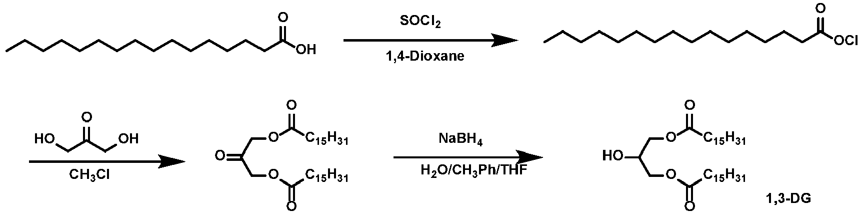

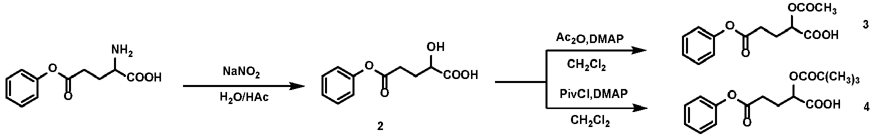

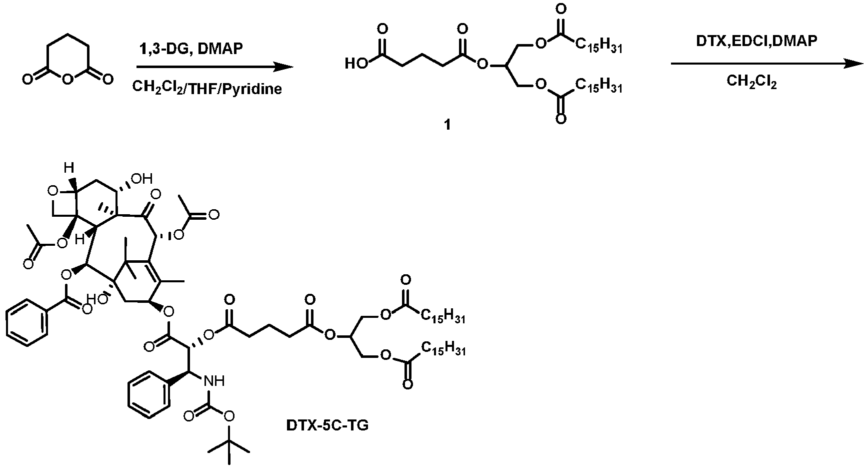

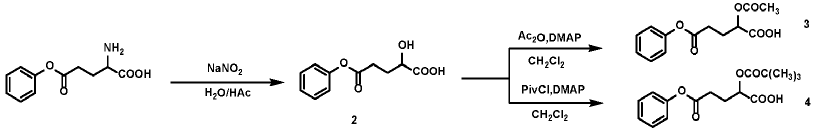

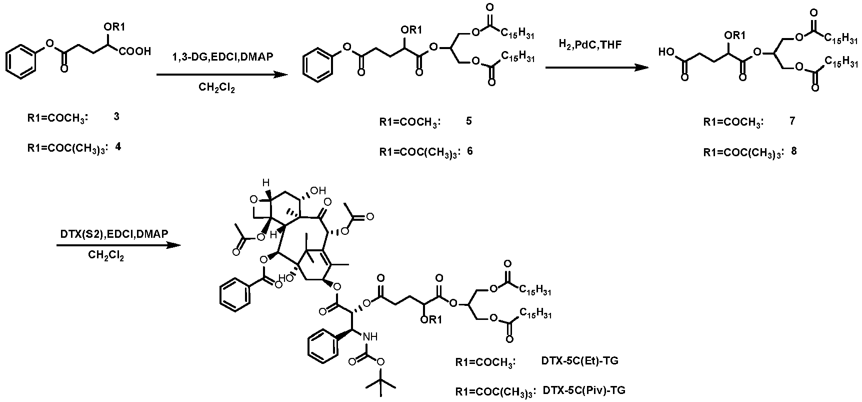

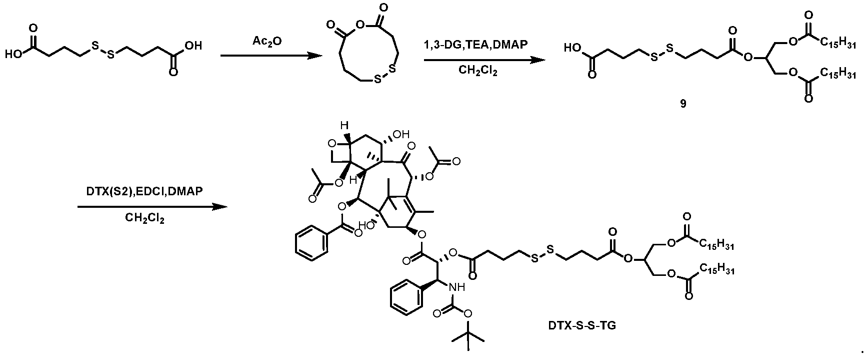

### Example 5 Preparation of chylomicron emulsions of docetaxel - triglyceride prodrugs

12 mg of docetaxel-triglyceride prodrug is dissolved in 400 mg of olive oil, and preheated to 60°C. 120 mg of egg yolk lecithin and 40 mg of sodium deoxycholate are weighed and dissolved in 4 ml of deionized water, and preheated to 60°C. An oil phase is slowly added dropwise to an aqueous phase under stirring, wherein the stirring is continued for 3 min to form colostrum; and the colostrum is subjected to ultrasonic treatment by a probe under an ice bath for 10 min, with an ultrasonic power of 500 W. The properties of formulations are shown in Table 1. The particle size of each of the four prodrug emulsions is about 215 nm, a zeta potential is about -40 mv, and an encapsulation rate is greater than 90%.

**Table 1. Properties of Triglyceride Prodrugs and Docetaxel Emulsion**

| Formulations | Size (nm) | PDI | Zeta potential (mv) | EE (%) |
|---|---|---|---|---|
| DTX Emulsion | 213±1.82 | 0.173±0.05 | -41.3±1.49 | 69.1±3.50 |
| DTX-5C-TG Emulsion | 204±1.25 | 0.188±0.02 | -36.5±3.84 | 93.0±0.63 |
| DTX-5C(Et)-TG Emulsion | 223±6.69 | 0.166±0.02 | -36.7±1.40 | 98.6±1.01 |
| DTX-S-S-TG Emulsion | 212±3.03 | 0.164±0.04 | -45.5±3.84 | 96.5±1.39 |
| DTX-5C(Piv)-TG Emulsion | 212±2.07 | 0.172±0.01 | -45.2±4.24 | 99.2±0.66 |

### Example 6 Stability of Docetaxel-Triglyceride Prodrug

Emulsions prepared in Example 5 are incubated in simulated gastric juice, bile-pancreatic juice simulated intestinal juice, and lipoprotein lipase-containing plasma at 37°C, respectively; and samples are taken at predetermined time points to investigate the physical stability of the prodrug emulsion and the chemical stability of the prodrug in different media. The stability results of the prodrug in the gastrointestinal tract and plasma are shown in FIG.s 1-2.

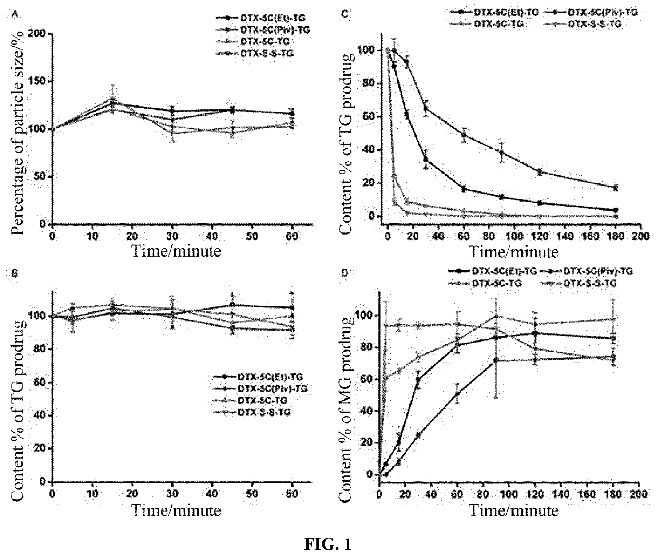

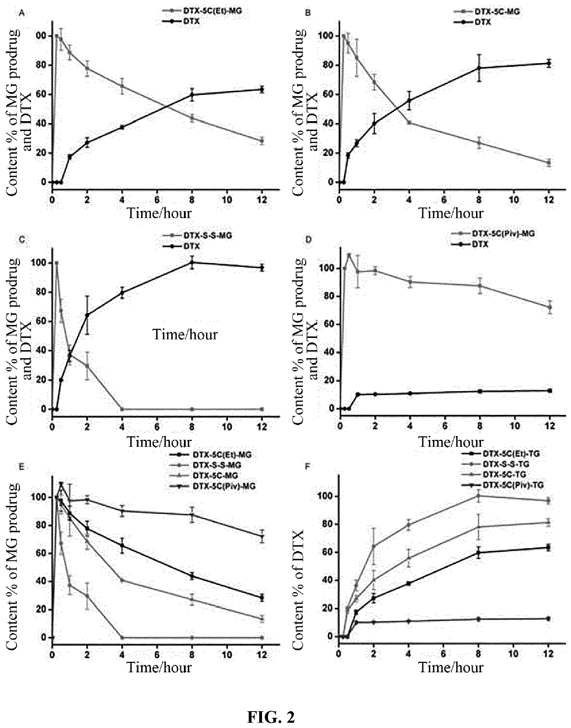

It can be seen from FIG. 1-A that the docetaxel-triglyceride prodrug emulsion maintains a stable particle size in simulated gastric juice within one hour, exhibiting good physical stability. It can be seen from FIG. 1-B that the docetaxel-triglyceride prodrug is not degraded in the simulated gastric juice within one hour. The prodrug shows good chemical stability. The above results indicate that the prodrug emulsion can resist the gastrointestinal environment without leakage of a formulation and without degradation either, and thus has good gastric stability. It can be seen from FIG.s 1-C and 1-D that 4 kinds of docetaxel-triglyceride prodrugs are degraded into docetaxel-monoglyceride prodrugs in intestinal juice, but degradation rates are different. DTX-5C(Et)-TG and DTX-5C(Piv)-TG respectively have a substituent at the α-position of a glyceride bond, which hinders the action of pancreatic lipase, resulting in a relatively slow degradation rate. However, DTX-5C-TG and DTX-S-S-TG have no α-steric hindrance, such that the activity of the pancreatic lipase is not affected, which is then quickly digested into monoglyceride prodrugs. In addition, DTX-S-S-TG has higher lipolysis efficiency due to its structure closer to the triglycerides in food. From the above results, we can infer that the lipolysis of lipase to the prodrugs is affected by a linking bridge structure. A straight linking bridge without a branched chain at α-position is more conducive to the lipolysis and digestion of pancreatic lipase to the triglyceride prodrugs in the intestine. Meanwhile, a longer straight linking bridge may be more advantageous. The results of plasma stability of the docetaxel-triglyceride prodrugs are shown in FIG. 2. Four kinds of prodrugs are rapidly degraded into docetaxel-monoglyceride prodrugs in plasma added with lipoprotein lipase. After continuous incubation, DTX-S-S-TG quickly releases a parent drug of docetaxel, and DTX-5C-TG and DTX-5C(Et)-TG release the drug slowly. However, DTX-5C(Piv)-TG hardly releases the docetaxel parent drug, since the steric hindrance of the branched chain of pivaloyl is too large, which affects the hydrolysis of esterase to an ester bond between docetaxel and aliphatic chains. From the results of plasma stability, it can be seen that the stability of the linking bridge has a great influence on the activation of the prodrug, and the introduction of a reduction-sensitive and cleavable dithio bond linking bridge is beneficial to the activation of the prodrug.

### Example 7 Reduction-sensitive release of docetaxel-triglyceride prodrugs

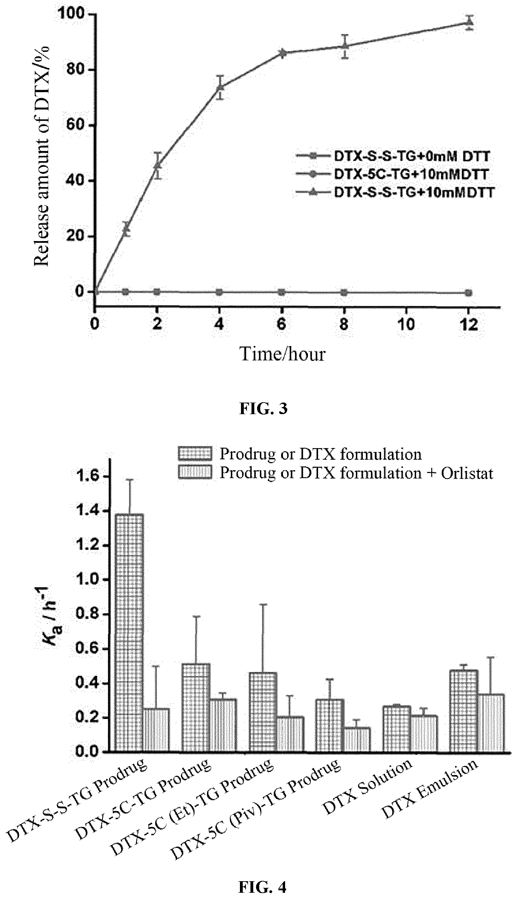

The release results of DTX-5C-TG and DTX-S-S-TG under in vitro reduction-sensitive (10 mmol DTT) or non-reduction-sensitive (0 mmol DTT) conditions are shown in FIG. 3. Under the non-reduction conditions, DTX-S-S-TG does not release a parent drug within 12 hours. Under the reduction conditions, DTX-5C-TG does not release the parent drug within 12 hours, while DTX-S-S-TG slowly releases completely. The above results indicate that DTX-S-S-TG can release the parent drug in a reduction environment, and thus can target tumor cells with high glutathione concentration and release the drug specifically.

### Example 8 Investigation of intestinal permeability of docetaxel-triglyceride prodrug

The intestinal permeability of a docetaxel-triglyceride prodrug is investigated by *in vivo* intestinal perfusion in rats. The results are shown in FIG. 4. Compared with a docetaxel solution, permeability coefficients *Kₐ* of a docetaxel-triglyceride prodrug emulsion DTX-S-S-TG prodrug, a DTX-5C-TG prodrug emulsion, a DTX-5C(Et)-TG prodrug emulsion, and a DTX-5C(Piv)-TG prodrug emulsion are increased by 5.13, 1.91, 1.73, and 1.15 times. In order to investigate the effect of intestinal pancreatic lipase digestion on the absorption of the prodrug, a pancreatic lipase inhibitor orlistat is administrated half an hour in advance. The results show that the pancreatic lipase inhibitor can significantly reduce the absorption of the prodrug, but has no significant effect on the absorption of a docetaxel solution. The above results indicate that the docetaxel-triglyceride prodrug can significantly improve the intestinal permeability of docetaxel. In addition, affected by the lipolysis of intestinal pancreatic lipase, more monoglycerides prodrug produced by high lipolysis efficiency are more conducive to the transmembrane absorption of docetaxel.

### Example 9 Pharmacokinetics of docetaxel-triglyceride prodrug

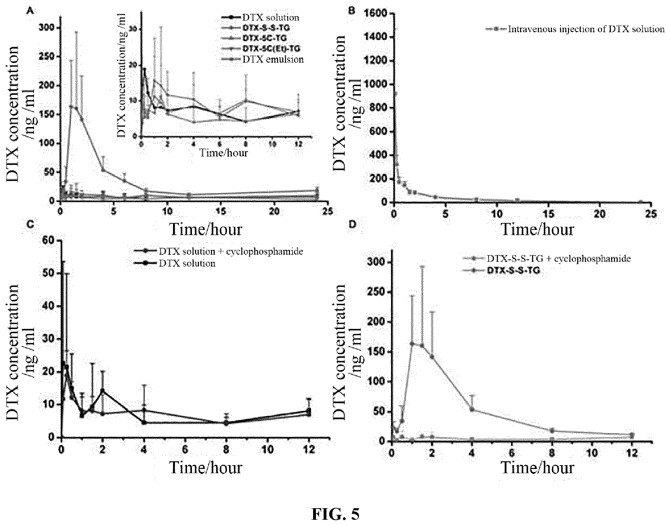

Taking SD rats as a model, a docetaxel-triglyceride prodrug emulsion and a docetaxel solution are orally administered; a plasma concentration of a parent drug is measured; and a drug-time curve is drawn and corresponding pharmacokinetic parameters are calculated respectively based on the measured concentration (Table 2). In order to calculate the absolute bioavailability, a docetaxel solution is administered intravenously, and the content of docetaxel in the plasma is measured. The drug-time curves of the prodrug and the parent drug are shown in FIG. 5-A. Compared with the docetaxel solution, the area under the drug-time curve of the docetaxel-triglyceride prodrug emulsion (AUC₀₋₂₄) is significantly larger than that of the docetaxel solution. The oral bioavailability of the prodrug is calculated from the data of intravenous docetaxel, and the oral bioavailability of each of DTX-5C-TG, DTX-5C(Et)-TG, and DTX-S-S-TG is significantly improved (improved by 1.05-4.71 times) than that of docetaxel (9.4%), indicating that the docetaxel-triglyceride prodrug significantly improves the oral absorption of docetaxel.

In addition, in order to investigate whether the docetaxel-triglyceride prodrug is transported through the lymphatics, one hour before oral administration of the DTX-S-S-TG and the docetaxel solution, cyclophosphamide is administered intraperitoneally to inhibit the release of chylomicrons. The effect of lymphatic transport on the absorption of the prodrug is investigated. The results are shown in FIGs. 5-C and 5-D. Compared with DTX-S-S-TG, the cyclophosphamide can significantly inhibit the oral absorption of DTX-S-S-TG. After lymphatic suppression, it is almost impossible to detect the plasma concentration of docetaxel, but there was no significant difference in AUC in the docetaxel solution before/after the administration of cyclophosphamide. It is indicated that the absorption pathway of the docetaxel-triglyceride prodrug is lymphatic transport. It is worth noting that the oral bioavailability of DTX-S-S-TG is significantly higher than that of the other three prodrugs, which is consistent with the excellent intestinal digestibility, good intestinal cell absorption and drug release characteristics in plasma exhibited by DTX-S-S-TG. However, due to steric hindrance, the lipolysis performance, membrane permeability and plasma release of DTX-5C(Piv)-TG are affected, resulting in the blood concentration thereof being lower than a detection limit.

**Table 2. Main pharmacokinetic parameters of docetaxel triglyceride prodrugs**

| Formulations | Tₘₐₓ(h) | Cₘₐₓ(ng/ml) | T_{1/2}(h) | AUC(0-24h) | Fᵣₑₗ % | Fabs % |
|---|---|---|---|---|---|---|
| DTX Solution | 0.4±0.5 | 21.0±6.8 | 11.8±8.4 | 167.8±125.0 | 100 | 9.4 |
| DTX Emulsion | 0.4±0.6 | 18.5±17.2 | 12.3±8.4 | 119.8±111.6 | 71.4 | 6.7 |
| DTX-5C-TG Emulsion | 2.8±3.2 | 18.9±4.8 | 12.5±4.0 | 176.7±101.3 | 105.3 | 9.9 |
| DTX-5C(Et)-TG Emulsion | 6.0±10.0 | 25.0±11.8 | 25.2±20.3 | 248.8±111.3 | 148.3 | 14 |
| DTX-S-S-TG Emulsion | 1.7±0.4 | 235.4±120.2 | 8.7±8.9 | 789.9±221.2 | 470.7 | 44.3 |

### Example 10 Cytotoxicity of docetaxel-triglyceride prodrug

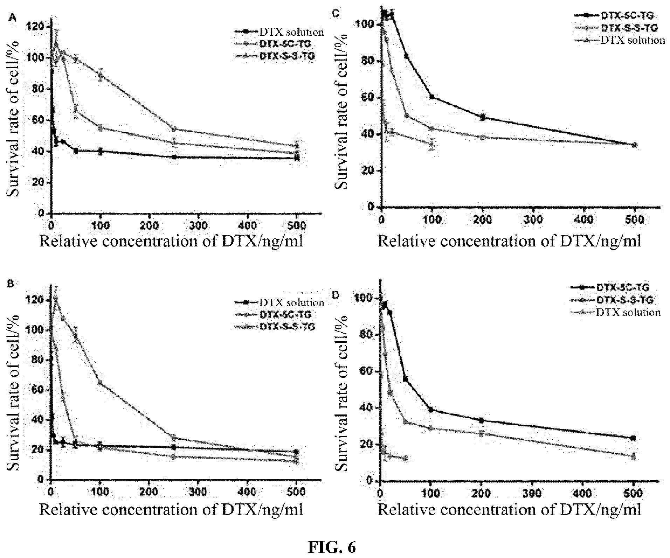

In order to investigate the tumor reduction-sensitive release of DTX-S-S-TG, 4T1 cells and KB cells are used as models to investigate the cytotoxicity of DTX-5C-TG, a DTX-S-S-TG emulsion and a docetaxel solution. A cell survival-concentration curve is shown in FIG. 6, and IC50 values are shown in Table 3. In the two cell models, DTX-S-S-TG shows stronger cytotoxicity than DTX-5C-TG, because both cells are tumor cells, with a high glutathione environment inside. Due to a reduction-sensitive dithio linking bridge, DTX-S-S-TG has higher drug release efficiency than DTX-5C-TG, which is linked by insensitive ester bonds. As a result, the cytotoxicity of DTX-S-S-TG is stronger than that of DTX-5C-TG. Meanwhile, due to the slow release of a sensitive prodrug, DTX-S-S-TG has lower cytotoxicity than the docetaxel solution.

**Table 3. IC₅₀ of docetaxel-triglyceride prodrug on cells**

| Formulations | 4T1 (ng/mL) | | KB (ng/mL) | |
|---|---|---|---|---|
| | 48 h | 72 h | 48 h | 72 h |
| DTX Solution | 16.6 | 5.9 | 4.9 | 1.3 |
| DTX-S-S-TG | 177.4 | 31.6 | 99.9 | 26.8 |
| DTX-5C-TG | 357.1 | 156.9 | 226.5 | 88.2 |

### Example 11 Pharmacodynamics of docetaxel-triglyceride prodrug

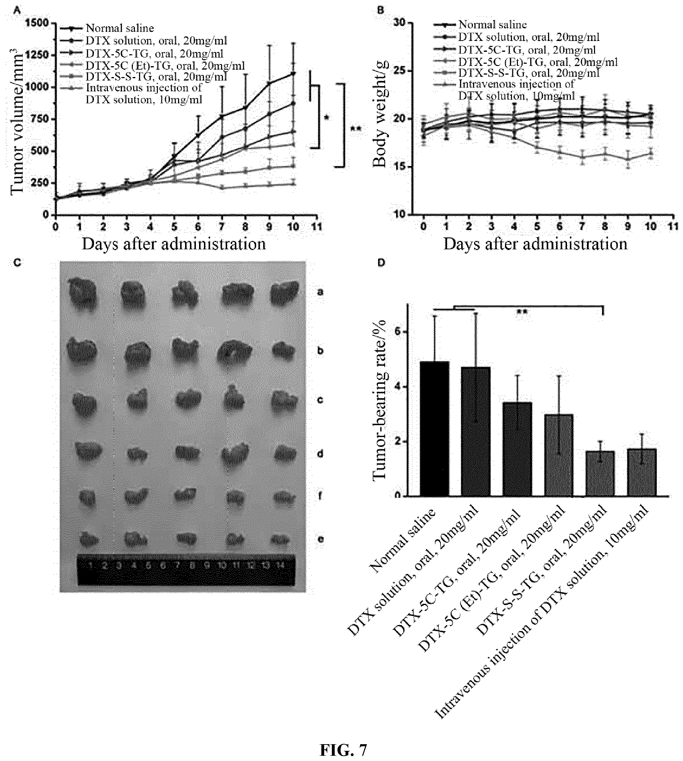

Taking 4T1 tumor-bearing Balb/c mice as a model, a docetaxel-triglyceride prodrug emulsion and a docetaxel solution are taken orally, and a docetaxel solution intravenous injection group is set as a positive control group. The results are shown in FIG. 7. There is no significant difference between an oral docetaxel solution group and a normal saline group, which have almost no anti-tumor effect. DTX-5C-TG and DTX-5C(Et)-TG both show certain anti-tumor effect. DTX-S-S-TG shows an anti-tumor effect equivalent to that of an intravenous docetaxel solution, without weight loss, indicating a good safety. The above results indicate that the docetaxel-triglyceride prodrug has an inhibitory effect on tumor growth, wherein the most significant prodrug is DTX-S-S-TG, which is consistent with the results of pharmacokinetics and cytotoxicity and cell release experiments. It reminds us that a good pharmacodynamic result not only requires a significant pharmacokinetic result, but also that a specific release at a tumor site is very important.

### Example 12 Gastrointestinal toxicity of docetaxel-triglyceride prodrug

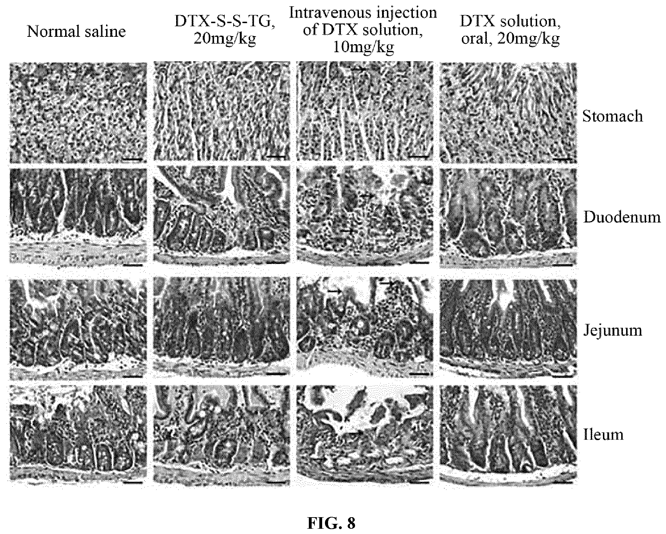

For 5 consecutive days, normal Balb/c mice are orally administered with the docetaxel-triglyceride prodrug emulsion synthesized in Example 4, and docetaxel solution, and intravenously injected with a docetaxel solution, and a normal saline group is used as control. The gastrointestinal toxicity is then investigated. The results are shown in FIG. 8. Intravenous injection of the docetaxel solution has an injury to respective intestinal segments. Among them, the jejunum and ileum have serious injuries such as apoptosis and shortening of villi. Oral administration of the docetaxel solution has slight injury to the jejunum, which may be due to its low-permeable membrane absorption. The docetaxel-triglyceride prodrug emulsion synthesized in Example 4 shows no injury to respective intestinal segments, which is consistent with the normal saline group. The above results indicate that the docetaxel-triglyceride prodrug has good gastrointestinal safety while improving the absorption.

## Claims

1. A compound represented by formula (I), (II), (III), geometric isomers, and pharmaceutically acceptable salts, hydrates and solvates thereof: 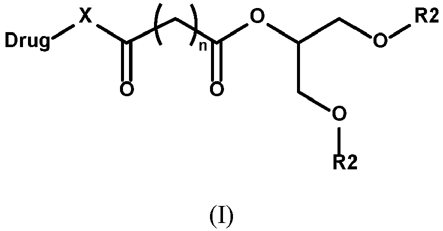 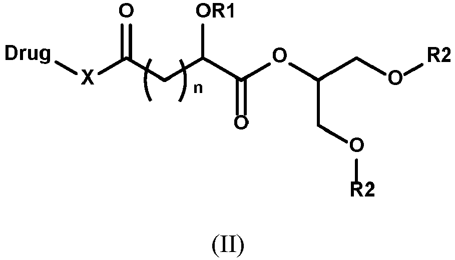 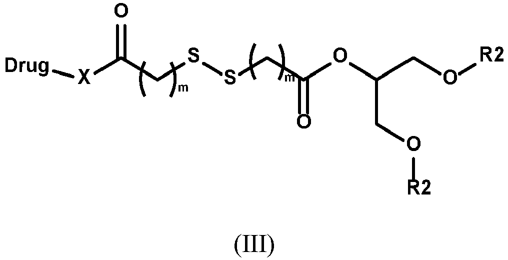
wherein X is NH, O, or S;
"Drug" is a poorly soluble drug containing hydroxyl, amino or sulfhydryl;
n=1-10; preferably 1-5; m=1-3;
R1 is C1-C6 acyl, preferably acetyl, propionyl, isopropionyl, or pivaloyl; and
R2 is C2-C24 saturated or unsaturated aliphatic acyl; preferably C10-C24 saturated or unsaturated aliphatic acyl.

2. The compound, geometric isomers, and pharmaceutically acceptable salts, hydrates, solvates, or prodrugs thereof according to claim 1, wherein
the poorly soluble drugs containing amino, hydroxyl or sulfhydryl comprise anti-tumor drugs, hormone drugs, hypolipidemic drugs, non-steroidal anti-inflammatory drugs and other poorly soluble drugs, wherein the anti-tumor drugs are selected from taxanes, anthraquinones, and nucleosides; the hormone drugs are selected from testosterone, and progesterone; the hypolipidemic drugs are statins; the non-steroidal anti-inflammatory drugs and other poorly soluble drugs are selected from halofantrine, griseofulvin, cyclosporine A and their derivatives.

3. The compound, geometric isomers, and pharmaceutically acceptable salts, hydrates, solvates, or prodrugs thereof according to claim 1 or 2, wherein

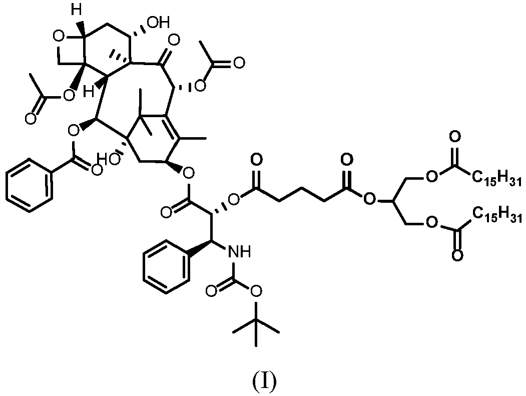

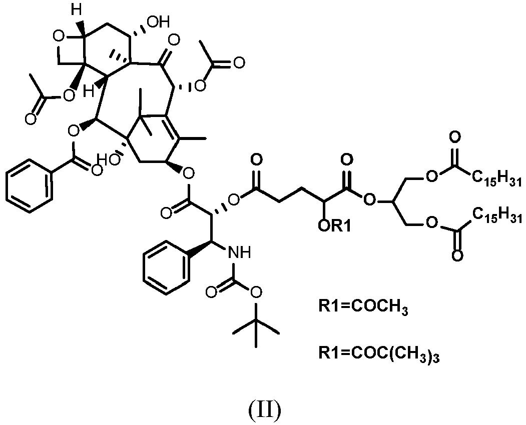

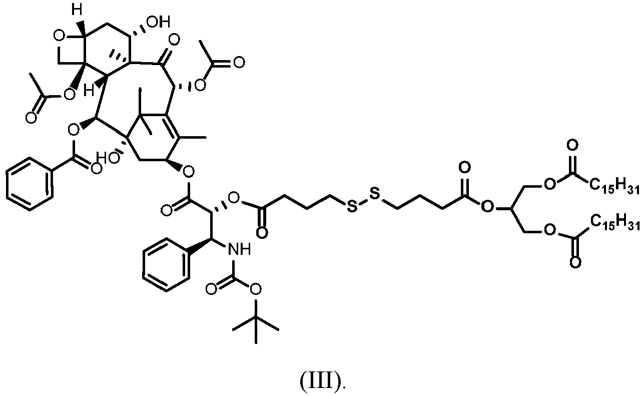

4. A method for preparing the compound according to claim 1, wherein the method for preparing the compound of general formula (I) includes:
(1) generating aliphatic acyl chloride from aliphatic acid in the presence of thionyl chloride; then reacting the aliphatic acyl chloride with 1,3-dihydroxyacetone under the catalyst of triethylamine to obtain a reactant; and preparing 1,3-diglyceride by catalytically hydrogenating the reactant with sodium borohydride; and
(2) reacting the 1,3-diglyceride with straight-chain aliphatic dibasic acid or aliphatic dibasic acid anhydride in the presence of pyridine to obtain a reactant; and generating the compound of general formula (I) by esterifying or ammoniating the reactant with an active drug in the presence of 4-dimethylaminopyridine.

5. The method for preparing the compound according to claim 1, wherein the method for preparing the compound of general formula (II) includes:
(1) generating aliphatic acyl chloride from the aliphatic acid in the presence of thionyl chloride; reacting the aliphatic acyl chloride with 1,3-dihydroxyacetone under the catalyst of triethylamine to obtain a reactant; and preparing 1,3-diglyceride by catalytically hydrogenating the reactant with sodium borohydride; and
(2) converting an amino group into a hydroxyl group by reacting L-glutamic acid-γ-benzyl ester with sodium nitrite in a mixed solvent of glacial acetic acid and water; esterifying with different alkanoyl chlorides or alkanoic acid anhydrides under the catalysis of 4-dimethylaminopyridine to obtain a product; esterifying the product and 1,3-diglyceride in the presence of DMAP and 1-(3-dimethylaminopropyl)-3-ethylcarbodiimide hydrochloride EDCI to obtain a product; catalytically reducing the product with hydrogen; and obtaining the compound of general formula (II) by performing an esterified or ammoniated reaction on the reduced product and an active drug in the presence of DMAP and EDCI.

6. The method for preparing the compound according to claim 1, wherein the method for preparing the compound of general formula (III) includes:
(1) generating aliphatic acyl chloride from the aliphatic acid in the presence of thionyl chloride; then reacting the aliphatic acyl chloride with 1,3-dihydroxyacetone under the catalyst of triethylamine to obtain a reactant; and preparing 1,3-diglyceride by catalytically hydrogenating the reactant with sodium borohydride; and
(2) reacting the 1,3-diglyceride with aliphatic dithio-dibasic acid anhydride in the presence of triethylamine to obtain a reactant; and obtaining the compound of general formula (III) by esterifying or ammoniating the reactant and an active drug in the presence of DMAP and EDCI.

7. The preparation method according to any one of the claims 4 to 6, wherein the aliphatic acid in step (1) is C2-C24 saturated or unsaturated aliphatic acid; the aliphatic dibasic acid in step (2) is selected from: malonic acid, succinic acid, glutaric acid, adipic acid, pimelic acid, suberic acid, azelaic acid, and sebacic acid; and the aliphatic dithio-dibasic acid anhydride is selected from: 2,2'-dithiodiacetic acid, 3,3'-dithiodipropionic acid, and 4,4'-dithiodibutyric acid.

8. The preparation method according to claim 5, wherein the L-glutamic acid-γ-benzyl ester can be replaced by 2-amino-3-benzylmalonic acid, 2-amino-4-benzyl succinic acid, 2-amino-6-benzyl adipic acid, 2-amino-7-benzyl pimelic acid, 2-amino-8-benzyl suberic acid, 2-amino-9-benzyl azelaic acid, and 2-amino-10-benzyl sebacic acid; and the alkanoyl chloride or alkanoic acid anhydride is selected from acetic acid anhydride, propionic acid chloride, isopropanoyl chloride, and pivaloyl chloride.

9. A pharmaceutical composition, comprising the compound, geometric isomers, and pharmaceutically acceptable salts, hydrates, solvates, or prodrugs thereof according to any one of claims 1 to 3, and a pharmaceutically acceptable carrier.

10. Use of the compound, geometric isomers, and pharmaceutically acceptable salts, hydrates, solvates, or prodrugs thereof according to any one of claims 1 to 3 or the pharmaceutical composition according to claim 9 in the preparation of anti-tumor drugs.

11. Use of the compound, geometric isomers, and pharmaceutically acceptable salts, hydrates, solvates, or prodrugs thereof according to any one of claims 1 to 3 or the pharmaceutical composition according to claim 9 in the preparation of targeted therapeutic drugs.
